# DEMANDE DE BREVET EUROPEEN

(11) **EP 4 825 416 A1**
(43) Date de publication de la demande: **21.10.2026**
(21) Numéro de dépôt: 25305551.1
(22) Date de dépôt: 15.04.2025
(51) Int. Cl.: A61M 5/28, A61M 5/31, A61M 5/315

(54) **SERINGUE POUR L'INJECTION D'UNE SOLUTION DESTINÉE À LA MÉDECINE ESTHÉTIQUE**

(71) Demandeur: Levy, Claude, 75016 Paris (FR)
(72) Inventeur: Levy, Claude, 75016 Paris (FR)
(74) Mandataire: Tranvouez, Edern Morgan

(57) **Abrégé**

L'invention concerne une seringue pour l'injection d'une solution destinée à la médecine esthétique, la seringue comportant un corps (20) s'étendant longitudinalement entre une première extrémité comportant une collerette (24) et une deuxième extrémité ; un piston (21) logé dans ledit corps (20) ; ledit piston (21) comporte une première extrémité ayant une tête de piston (23) et une deuxième extrémité d'appui, la seringue étant remarquable en ce que le piston (21) comporte : au moins une première face (25), une deuxième face (26) et une troisième face (27) ; ladite première face (25) et ladite deuxième face (26) comportent une surface dentée ; ladite troisième face (27) comporte une surface lisse ; la collerette (24) comprend une lame (22) dont une extrémité est en prise avec les dents de la première face (25) ou de la deuxième face (26) lorsque ladite face est agencée en regard de la lame (22).

## Description

### Domaine technique de l'invention

La présente invention concerne une seringue pour l'injection d'une solution destinée à la médecine esthétique, notamment d'une solution de toxines du type botulique. Elle s'applique, dans le domaine de la médecine esthétique, l'ophtalmologie et la neurologie.

### Technique antérieure

La seringue est un dispositif utilisé en médecine pour administrer ou prélever des substances liquides. Elle se compose d'un cylindre gradué, d'un piston ajusté et d'une aiguille creuse. L'opérateur remplit la seringue en aspirant la solution à injecter ou le fluide à recueillir, puis exerce une pression pour libérer la substance. La substance à injecter peut nécessiter une manipulation rigoureuse et précise.

Dans le cadre de la médecine esthétique, utilisant notamment une solution de toxines du type botulique. La seringue doit être adaptée au bon dosage des toxines pour injecter une solution suffisante pour la pathologie du patient.

L'injection de toxine botulique en médecine esthétique nécessite une précision absolue pour garantir l'efficacité du traitement et éviter les effets secondaires indésirables. L'un des défis majeurs pour le praticien est le ciblage précis des muscles à traiter. Une injection mal dosée peut conduire une diffusion inappropriée du produit, avec pour conséquences un résultat disgracieux, une asymétrie faciale, voire des complications fonctionnelles.

Lors de la préparation de la toxine botulique avant son injection. La grande majorité des toxines disponibles sur le marché sont lyophilisées et nécessitent une reconstitution avec du sérum physiologique avant leur administration. Ce processus implique l'utilisation de seringues adaptées aux volumes nécessaires pour obtenir la concentration souhaitée. Or, si une seringue de 1 ml est adaptée pour reconstituer l'Azzalure^{™} qui nécessite 0,64 ml de sérum physiologique ou utiliser l'Alluzience^{™}, il n'existe actuellement aucune seringue permettant une reconstitution simple des autres toxines tel que le Vistabel^{™}, Bocouture^{™}, ou Letybo^{™} qui nécessitent 1,25 ml de sérum physiologique ou utiliser le Relfydess^{™}

Cette absence d'un dispositif adéquat oblige les praticiens à utiliser des seringues de 1ml, ce qui pose un problème pour la précision d'injection, ou à effectuer des manipulations supplémentaires pour obtenir le bon mélange, ce qui augmente le risque d'erreur, de contamination et de perte de produit.

De plus, quelle que soit la toxine choisie, le praticien doit être précis dans la dose de toxine à injecter, celle -ci variant de 0,0125 ml à 0,05 voire 0,1 ml en fonction du type de toxine. Sans indicateur lors de l'injection, cette précision n'est pas aisée à atteindre.

Afin de faciliter ce dosage, il est connu du document de publication EP2821089 A1 une seringue à graduations alternativement sélectionnables offrant un indicateur tactile et auditif sur les doses injectées.

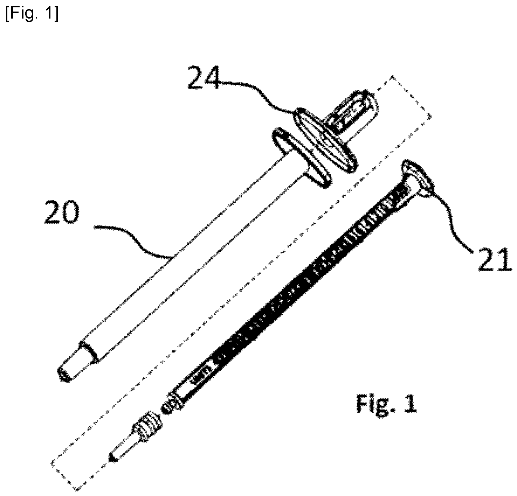

Cet exemple de réalisation de l'art antérieur d'une telle seringue est représenté sur la figure 1.

La figure 1 montre une seringue comportant un corps et un piston.

L'une des extrémités du corps comporte une collerette.

Une partie du piston se loge dans le corps à partir de la première extrémité. Le piston présente une section latérale polygonale dont chacune des arrêtes est munies de rainures avec le pas de rainures correspondant une dose à injecter de toxine.

Cette seringue comprend en outre un mécanisme qui équipe la collerette et qui comprend une lame venant s'engager avec des rainures aménagées sur les arrêtes du piston et un mécanisme de verrouillage permettant de verrouiller ou de libérer l'engagement de la lame avec les rainures des arrêtes du piston. Ainsi, lors du remplissage de la seringue, le praticien doit déverrouiller la lame afin de permettre un déplacement libre du piston alors que lors de l'injection, en outre du réglage de la dose par la rotation du piston, le praticien doit penser à verrouiller la lame afin de retrouver le retour tactile et auditif pour chaque dose injectée.

Toutefois, cette seringue repose sur un mécanisme complexe. Lorsque la pathologie du patient nécessite une toxine précise, le remplissage de la seringue ou la bonne configuration de la seringue demande plusieurs actions. De plus ce mécanisme requière un nombre important de pièces qui se répercute sur les coûts de fabrication de la seringue.

### Présentation de l'invention

La présente invention vise à remédier à ces inconvénients avec une approche totalement novatrice.

Ces objectifs, ainsi que d'autres qui apparaîtront par la suite, sont atteints, selon un premier aspect, à l'aide d'une seringue pour l'injection d'une solution destinée à la médecine esthétique, notamment d'une solution de toxines du type botulique, la seringue ayant un axe longitudinal et comportant :
- un corps de forme cylindrique circulaire creux s'étendant longitudinalement entre une première extrémité comportant une collerette et une deuxième extrémité d'injection destiné à être équipée d'une aiguille ;
- un piston de forme longitudinale logé dans ledit corps ; ledit piston comporte une première extrémité ayant une tête de piston et une deuxième extrémité d'appui,

la seringue étant remarquable en ce que le piston comporte :
   - au moins une première face longitudinale, une deuxième face longitudinale et une troisième face longitudinale ; ladite première face longitudinale et ladite deuxième face longitudinale comportent une surface dentée et différente l'une de l'autre ; ladite troisième face comporte une surface lisse ; les dents de la première et de la deuxième surface étant espacées régulièrement pour correspondre à un volume prédéfini respectif de solution à injecter ;
en ce que la collerette comprend une lame dont une extrémité est en prise avec respectivement les dents de la première face longitudinale ou de la deuxième face longitudinale lorsque ladite face longitudinale du piston est agencée en regard de la lame,
et en ce que le corps est configuré pour contenir un volume d'un contenant compris entre 1,3 ml et 1,7 ml.

Grâce à ces dispositions, la seringue permet de proposer un dosage précis sans avoir de mécanisme complexe nécessitant plusieurs actions et coûteux à fabriquer.

Les première et deuxième face sont pourvues de surfaces dentées différentes, dont l'espacement régulier des dents permet de prédéfinir des volumes d'injection. Cette différenciation offre au praticien une flexibilité accrue dans le choix du dosage, garantissant ainsi une administration précise et reproductible de la solution injectable.

En sélectionnant l'une ou l'autre de ces faces, l'utilisateur peut adapter la seringue aux besoins spécifiques du traitement et du patient, tout en réduisant le risque d'erreur de dosage.

La troisième face longitudinale lisse apporte une configuration alternative au mode de fonctionnement cranté. Cette face permet un déplacement fluide du piston, sans résistance ceci notamment pour le remplissage. Cette caractéristique est particulièrement utile lorsque le praticien souhaite ajuster manuellement le volume de toxine à prélever avant son injection, notamment dans le cadre de la reconstitution de la solution de toxine avec du sérum physiologique.

Les injections de toxine botulique sont plus efficaces et mieux contrôlées. Elles garantissent des résultats esthétiques optimaux et satisfaisant à la fois le praticien et le patient.

La fonction de la lame agit comme un système de contrôle du déplacement du piston. Cela permet d'obtenir un dosage précis de la solution injectable. L'existence de dents offre un retour tactile et auditif au praticien lui permettant de mieux maîtriser l'injection et de limiter les risques de mouvements involontaires du piston.

Ainsi le praticien bénéficie, avec la présente seringue,d'un suivi précis de l'injection.

L'invention est avantageusement mise en œuvre selon les modes de réalisation et les variantes exposées ci-après, lesquelles sont à considérer individuellement ou selon toute combinaison techniquement opérante.

Dans un mode de réalisation, l'extrémité d'appui du piston et de la collerette comprend pour chacune des faces longitudinales du piston un indicateur respectif s'alignant avec un indicateur de l'autre de l'extrémité d'appui du piston et de la collerette lorsque la face correspondante est positionnée en regard de la lame.

Dans un mode de réalisation, c'est la collerette qui comprend l'indicateur respectif pour chacune des faces longitudinales du piston qui s'aligne avec un indicateur de l'extrémité d'appui du piston et dans lequel l'indicateur de l'extrémité d'appui est une ouverture ménagée dans ladite extrémité par laquelle ledit indicateur respectif est visible lorsque la face correspondante est positionnée en regard de la lame.

Grâce à ces dispositions, l'indicateur permet à l'utilisateur de la seringue de connaître quelle face du piston il va utiliser afin de vérifier qu'elle correspond à la solution qu'il va injecter.

Dans un mode de réalisation, la collerette comprend un élément support formé monobloc avec le reste du corps et un élément d'habillage agencé autour de l'élément de corps et dans lequel la lame est logée entre l'élément support et l'élément d'habillage.

Dans un mode de réalisation, l'élément d'habillage comprend les indicateurs respectifs.

Grâce à ces dispositions, les indicateurs permettent de s'assurer que c'est la bonne face piston qui est agencé en face de la lame et donc que le retour tactile et auditif correspond bien au dosage attendu.

Dans un mode de réalisation, la première face et la deuxième face comporte un pas de dents correspondant à la distance entre deux dents de ladite face ; ledit pas de dents d'une des faces correspond au moins au double du pas de dents de l'autre face.

Grâce à ces dispositions, le volume de la solution à injecter sera précis et nécessitant pas d'autres actions.

Dans un mode de réalisation, le pas de dents de la première face correspond à un volume de solution compris entre 0,0300 ml et 0,0050 ml, voire entre 0,0150 ml et 0,0100 ml et de manière particulièrement avantageuse égale à 0,0125 ml, et ledit pas de dents de la deuxième face correspond à un volume compris entre 0,0400 ml et 0,0100 ml, voire entre 0,0300 ml et 0,0200 ml et de manière particulièrement avantageuse égale à 0,0250 ml.

Dans un mode de réalisation, le piston comporte, préférentiellement, une quatrième face ayant une surface lisse.

Selon un deuxième aspect, la présente invention vise un procédé de fabrication d'une seringue pour l'injection d'une solution destinée à la médecine esthétique, remarquable en ce qu'elle comporte les étapes suivantes :
- a) insérer une lame sur une collerette appartenant à un corps de forme cylindrique circulaire creux s'étendant longitudinalement entre une première extrémité comprenant ladite collerette et une deuxième extrémité d'injection destiné à être équipée d'une aiguille ;
- b) insérer un piston de forme longitudinale dans le corps ; ledit piston comporte une première extrémité ayant une tête de piston et une deuxième extrémité d'appui ; le piston comporte une première face longitudinale, une deuxième face longitudinale et une troisième face longitudinale ; ladite première face longitudinale et ladite deuxième face longitudinale comportent une surface dentée et différente l'une de l'autre ; ladite troisième face comporte une surface lisse ; l'une des extrémité de ladite lame est en prise avec respectivement les dents de la première face longitudinale ou de la deuxième face longitudinale lorsque ladite face longitudinale du piston est agencée en regard de la lame.

Bien entendu, les étapes a) et b) peuvent être inversées, sans que l'on sorte du cadre de l'invention.

Les avantages, buts et caractéristiques particulières de ce procédé étant similaires à ceux de la seringue objet de la présente invention, ils ne sont pas rappelés ici.

Selon un troisième aspect, la présente invention vise un procédé de remplissage d'une seringue dans laquelle il est prévu les étapes suivantes :
- 1) placer ladite troisième face du piston en regard de la lame ;
- 2) remplir la seringue à partir d'une solution à injecter, la lame laissant le piston libre de se déplacer pendant ce remplissage, le corps étant configuré pour contenir un volume d'un contenant compris entre 1,3 ml et 1,7 ml ;
- 3) placer la première face ou la deuxième face en regard de la lame de telle manière que les dents de ladite face soient en prise avec ladite lame.

Les avantages, buts et caractéristiques particulières de ce procédé étant similaires à ceux de la seringue objet de la présente invention, ils ne sont pas rappelés ici.

### Brève description des figures

D'autres avantages, buts et caractéristiques de la présente invention ressortent de la description qui suit, faite dans un but explicatif et nullement limitatif, en regard des dessins annexés, dans lesquels :
La figure 1 (relative à l'art antérieur) représente une vue en coupe d'une seringue ;
La figure 2 représente une vue en coupe montrant l'intérieur d'une seringue ;
La figure 3 représente une vue locale en coupe sur la première extrémité du corps ;
La figure 4 représente une vue éclatée de la seringue ;
La figure 5 représente une vue isométrique de la seringue.

### Description des modes de réalisation

La figure 1 montre un schéma relatif à l'art antérieur d'une vue en coupe d'une seringue.

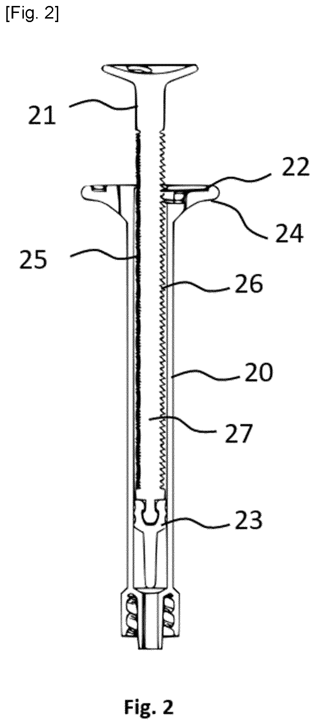

La figure 2 montre une vue en coupe montrant l'intérieur d'une seringue.

La vue en coupe montre les éléments de la seringue, il comporte : un corps 20, un piston 21, et une collerette 24.

Le corps 20 comporte une première extrémité et une deuxième extrémité.

La première extrémité comprend la collerette 24 et la deuxième extrémité destinée à être équipée d'une aiguille.

Selon un exemple de réalisation, le corps 20 comporte une forme cylindrique circulaire creux.

Le corps 20 comporte une longueur, et un diamètre. La longueur est comprise entre 60 mm et 100 mm. Selon une variante, la longueur est de 80 mm. Le diamètre est compris entre 6 mm et 11 mm. Selon une variante, le diamètre est de 8 mm.

La première extrémité comporte une épaisseur comprise entre 3 mm et 7 mm. Selon une variante, l'épaisseur est de 5 mm.

La deuxième extrémité comporte un diamètre comprise entre 6 mm et 12 mm. Selon un exemple, le diamètre est de 10 mm.

Selon un exemple réalisé, le corps 20 est configuré pour contenir un volume d'un contenant compris entre 1,3 ml et 1,7 ml.

La seringue comporte un axe longitudinal et le corps 20 s'étend longitudinalement.

Le corps 20 est monobloc, cela permet de réduire le nombre de pièce nécessaire à la fabrication de la seringue.

La collerette 24 comporte un élément support monobloc avec le reste du corps 20, et un élément d'habillage agencé autour du corps 20.

Le piston 21 comporte une forme longitudinale se logeant dans le corps 20.

Le piston 21 est inséré dans le corps 20 par la première extrémité.

Selon un exemple réalisé, le piston 21 comporte une première extrémité ayant une tête de piston 23 et une deuxième extrémité d'appui.

La première extrémité du piston 21 est logée dans le corps 20.

La deuxième extrémité d'appui correspond à un bouton poussoir permettant à un utilisateur d'appuyer sur le piston 21 pour l'enfoncer dans le corps 20.

Le piston 21 comporte, préférentiellement, une section triangulaire.

Selon une variante, la section est carrée.

Selon l'exemple réalisé avec la section triangulaire, le piston 21 comprend une première face 25 longitudinale, une deuxième face 26 longitudinale, et une troisième face 27 longitudinale.

La première face 25 et la deuxième face 26 comporte une surface dentée différente l'une de l'autre.

La troisième face 27 comporte une surface lisse.

Les dents des surfaces dentées de la première face 25 et de la deuxième face 26 sont espacées permettant de mettre un volume prédéfini respectif de solution à injecter.

La surface dentée de la première face 25 et de la deuxième face 26 comporte un pas de dents.

Ce pas de dents correspond à la distance entre deux dents d'une face.

Selon un exemple réalisé, le pas de dents d'une des faces correspond au moins au double du pas de dents de l'autre face.

Le corps comporte une lame 22 bloquée par la collerette 24, dont une extrémité libre est en contact avec une des faces du piston 21.

La lame 22 est logée entre l'élément support et l'élément d'habillage.

L'une des extrémités de la lame 22 est en prise avec les dents de la première face 25 ou de la deuxième face 26 lorsque l'une de ces faces est agencée en regard de la lame 22.

Le piston 21 comporte une course de piston 21 comprise entre 30 mm et 80 mm. Selon un exemple, la course du piston 21 est de 60 mm.

Lors de l'ajout de la collerette 24, le corps 20 comporte des ergots permettant de maintenir la collerette 24.

Naturellement, l'invention est décrite dans ce qui précède à titre d'exemple. Il est entendu que l'homme du métier est à même de réaliser différentes variantes de réalisation de l'invention sans pour autant sortir du cadre de l'invention.

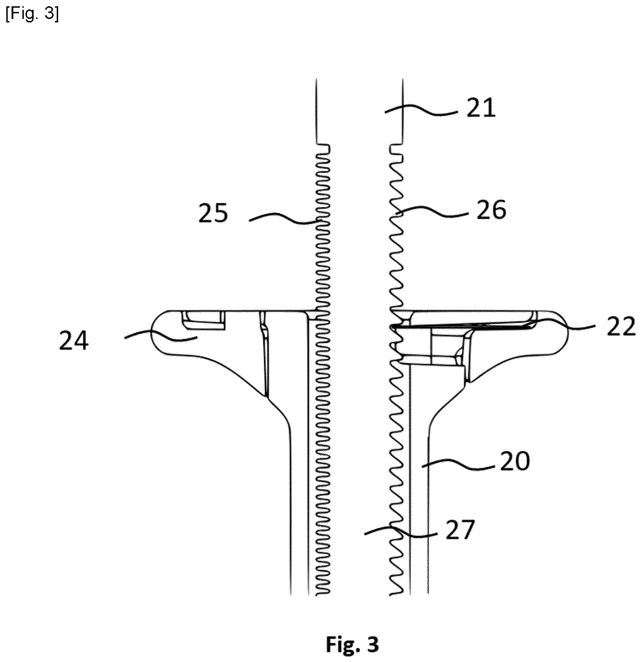

La figure 3 montre une vue locale en coupe sur la première extrémité du corps.

Le pas de dents de la première face 25 correspond à un volume de solution compris entre 0,0300 ml et 0,0050 ml, voire entre 0,0150 ml et 0,0100 ml.

Selon un exemple, le pas de dents de la première face 25 correspond à un volume de solution, de manière particulièrement avantageuse, égale à 0,0125 ml.

Le pas de dents de la deuxième face 26 correspond à un volume compris entre 0,0400 ml et 0,0100 ml, voire entre 0,0300 ml et 0,0200 ml.

Selon un exemple, le pas de dents de la deuxième face 26 correspond à un volume de solution, de manière particulièrement avantageuse, égale à 0,0250 ml.

La lame 22 est faite en matériau flexible permettant le bon contact avec les dents de la première face 25 ou de la deuxième face 26 tout en restant dans l'élément de support et l'élément d'habillage de la collerette 24.

Selon une variante réalisée, le piston 21 comporte une quatrième face comprenant une surface lisse.

Cette quatrième face est positionnée à l'opposé de la troisième face 27 dans le corps 20.

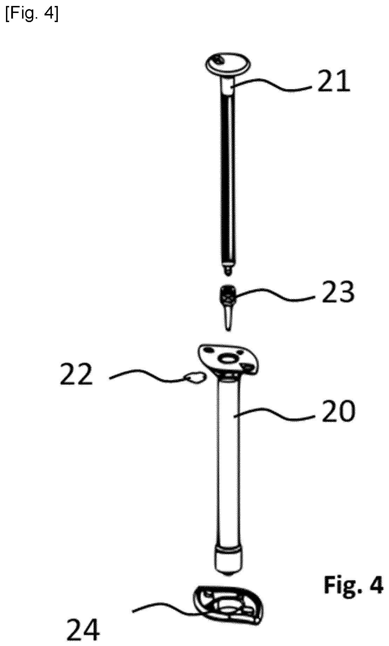

La figure 4 montre une vue éclatée de la seringue.

L'extrémité d'appui du piston 21 et de la collerette 24 comprend un indicateur pour chacune des faces longitudinales du piston 21.

Cet indicateur s'aligne avec un indicateur d'alignement de l'autre de l'extrémité d'appui du piston 21 et de la collerette 24 lorsque la face correspondante est positionnée en regard de la lame 22.

La collerette 24 comporte l'indicateur respectif pour chacune des faces longitudinales du piston 21 qui s'aligne avec un indicateur d'alignement de l'extrémité d'appui du piston 21.

L'indicateur d'alignement de l'extrémité d'appui est une ouverture ménagée dans ladite extrémité par laquelle ledit indicateur respectif de la collerette est visible lorsque la face correspondante est positionnée en regard de la lame 22.

Cette figure montre également un procédé de fabrication d'une seringue.

Ce procédé comporte deux étapes.

La première étape montre une insertion de la lame 22 et l'insertion de la collerette 24 par le bas (deuxième extrémité du corps 20).

La deuxième étape parle de l'insertion du piston 21 dans le corps 20.

En variante, les première et deuxième étapes peuvent être inversée, l'insertion du piston 21 étant alors préalable à l'insertion de la lame 22. Dans ce cadre là, la lame 22 est insérée par le côté et la collerette 24 par les bas. De cette manière, on évite que la lame n'abime la tête du piston 23.

Avant la deuxième étape, il y a une étape d'assemblage de la tête du piston 23 avec le piston 21.

Selon une variante, la deuxième étape d'insertion du piston 21 est réalisé avant la première étape d'insertion de la lame 22.

Cela permet d'éviter à la lame 22 d'abimer la seringue.

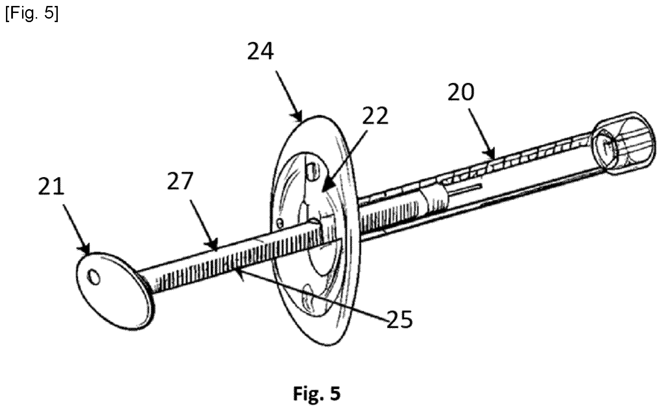

La figure 5 représente une vue isométrique de la seringue.

Cette figure permet également de montrer le procédé de remplissage de la seringue.

La première étape consiste à placer la troisième face 27 en regard de la lame 22. La troisième face 27 permet de contenir un volume supérieur ou égale à la première face 25 ou à la deuxième face.

La deuxième étape consiste à remplir la seringue à partir d'une solution à injecter.

Selon un exemple, la solution est une toxine du type botulique.

Une solution de toxine du type botulique est une substance permettant de détendre les muscles pour lisser les rides ou traiter certains problèmes médicaux comme les spasmes musculaires ou la transpiration excessive.

Pendant le remplissage, la lame 22 laisse le piston 21 en mouvement libre.

Le corps 20 comporte des graduations permettant de savoir approximativement le volume de contenu à injecter.

Les graduations sont positionnées entre la collerette 24 et la deuxième extrémité d'injection du corps 20.

La dernière étape consiste à placer la première face 25 ou la deuxième face 26 en regard de la lame 22.

Les dents de la première face 25 ou de la deuxième face sont prises avec la lame 22.

Il est souligné que toutes les caractéristiques, telles qu'elles se dégagent pour un homme du métier à partir de la présente description, des dessins et des caractéristiques attachées, même si concrètement elles n'ont été décrites qu'en relation avec d'autres caractéristiques déterminées, tant individuellement que dans des combinaisons quelconques, peuvent être combinées à d'autres caractéristiques ou groupes de caractéristiques divulguées ici, pour autant que cela n'ait pas été expressément exclu ou que des circonstances techniques rendent de telles combinaisons impossibles ou dénuées de sens.

### LISTE DES SIGNES DE RÉFÉRENCE

**[Table 1]**

| Références | Désignations |
|---|---|
| 20 | Corps |
| 21 | Piston |
| 22 | Lame |
| 23 | Tête de piston |
| 24 | Collerette |
| 25 | Première face |
| 26 | Deuxième face |
| 27 | Troisième face |

## Revendications

1. Seringue pour l'injection d'une solution destinée à la médecine esthétique, notamment d'une solution de toxines du type botulique, la seringue ayant un axe longitudinal et comportant :
- un corps (20) de forme cylindrique circulaire creux s'étendant longitudinalement entre une première extrémité comportant une collerette (24) et une deuxième extrémité d'injection destiné à être équipée d'une aiguille ;
- un piston (21) de forme longitudinale logé dans ledit corps (20) ; ledit piston (21) comporte une première extrémité ayant une tête de piston et une deuxième extrémité d'appui,
la seringue étant **caractérisée en ce que** le piston (21) comporte :
- au moins une première face (25) longitudinale, une deuxième face (26) longitudinale et une troisième face (27) longitudinale ; ladite première face (25) longitudinale et ladite deuxième face (26) longitudinale comportent une surface dentée et différente l'une de l'autre ; ladite troisième face (27) comporte une surface lisse ; les dents de la première et de la deuxième surface étant espacées régulièrement pour correspondre à un volume prédéfini respectif de solution à injecter ;
**en ce que** la collerette (24) comprend une lame (22) dont une extrémité est en prise avec respectivement les dents de la première face (25) longitudinale ou de la deuxième face (26) longitudinale lorsque ladite face longitudinale du piston (21) est agencée en regard de la lame (22),
et **en ce que** le corps (20) est configuré pour contenir un volume d'un contenant compris entre 1,3 ml et 1,7 ml.

2. Seringue selon la revendication 1, dans laquelle l'extrémité d'appui du piston (21) et de la collerette (24) comprend pour chacune des faces longitudinales du piston (21) un indicateur respectif s'alignant avec un indicateur de l'autre de l'extrémité d'appui du piston (21) et de la collerette (24) lorsque la face correspondante est positionnée en regard de la lame (22).

3. Seringue selon la revendication 2, dans laquelle c'est la collerette (24) qui comprend l'indicateur respectif pour chacune des faces longitudinales du piston (21) qui s'aligne avec un indicateur de l'extrémité d'appui du piston (21) et dans lequel l'indicateur de l'extrémité d'appui est une ouverture ménagée dans ladite extrémité par laquelle ledit indicateur respectif est visible lorsque la face correspondante est positionnée en regard de la lame (22).

4. Seringue selon l'une quelconque des revendications 1 à 3, dans laquelle, la collerette (24) comprend un élément support formé monobloc avec le reste du corps (20) et un élément d'habillage agencé autour de l'élément de corps (20) et dans lequel la lame (22) est logée entre l'élément support et l'élément d'habillage.

5. Seringue selon les revendications 3 et 4 dans laquelle l'élément d'habillage comprend les indicateurs respectifs.

6. Seringue selon l'une quelconque des revendications 1 à 5, dans laquelle la première face (25) et la deuxième face (26) comporte un pas de dents correspondant à la distance entre deux dents de ladite face ; ledit pas de dents d'une des faces correspond au moins au double du pas de dents de l'autre face.

7. Seringue selon l'une quelconque des revendications 1 à 6, dans lequel le pas de dents de la première face (25) correspond à un volume de solution compris entre 0,0300 ml et 0,0050 ml, voire entre 0,0150 ml et 0,0100 ml et de manière particulièrement avantageuse égale à 0,0125 ml, et ledit pas de dents de la deuxième face (26) correspond à un volume compris entre 0,0400 ml et 0,0100 ml, voire entre 0,0300 ml et 0,0200 ml et de manière particulièrement avantageuse égale à 0,0250 ml.

8. Seringue selon l'une des revendications 1 à 7, dans lequel le piston (21) comporte, préférentiellement, une quatrième face ayant une surface lisse.

9. Procédé de fabrication d'une seringue selon l'une des revendications 1 à 8, pour l'injection d'une solution destinée à la médecine esthétique, **caractérisé en ce qu'**elle comporte les étapes suivantes :
- a) insérer une lame (22) sur une collerette (24) appartenant à un corps (20) de forme cylindrique circulaire creux s'étendant longitudinalement entre une première extrémité comprenant ladite collerette (24) et une deuxième extrémité d'injection destiné à être équipée d'une aiguille ;
- b) insérer un piston (21) de forme longitudinale dans le corps (20) ; ledit piston (21) comporte une première extrémité ayant une tête de piston (23) (21) et une deuxième extrémité d'appui ; le piston (21) comporte une première face (25) longitudinale, une deuxième face (26) longitudinale et une troisième face (27) longitudinale ; ladite première face (25) longitudinale et ladite deuxième face (26) longitudinale comportent une surface dentée et différente l'une de l'autre ; ladite troisième face (27) comporte une surface lisse ; l'une des extrémité de ladite lame (22) est en prise avec respectivement les dents de la première face (25) longitudinale ou de la deuxième face (26) longitudinale lorsque ladite face longitudinale du piston (21) est agencée en regard de la lame (22).

10. Procédé de remplissage d'une seringue selon l'une quelconque des revendications 1 à 8, dans laquelle il est prévu les étapes suivantes :
- 1) placer ladite troisième face (27) du piston (21) en regard de la lame (22) ;
- 2) remplir la seringue à partir d'une solution à injecter, la lame (22) laissant le piston (21) libre de se déplacer pendant ce remplissage, le corps (20) étant configuré pour contenir un volume d'un contenant compris entre 1,3 ml et 1,7 ml ;
- 3) placer la première face (25) ou la deuxième face (26) en regard de la lame (22) de telle manière que les dents de ladite face soient en prise avec ladite lame (22).
